(19) **Europäisches Patentamt** / **European Patent Office** / **Office européen des brevets**

(11) **EP 3 607 870 B1**

(12) **EUROPEAN PATENT SPECIFICATION**

(45) Date of publication and mention of the grant of the patent:
**24.02.2021 Bulletin 2021/08**

(21) Application number: **18840618.5**

(22) Date of filing: **03.08.2018**

(51) Int Cl.:
*A61B 1/045* (2006.01)    *A61B 1/00* (2006.01)
*G02B 23/24* (2006.01)    *A61B 5/06* (2006.01)

(86) International application number:
**PCT/JP2018/029160**

(87) International publication number:
**WO 2019/027031 (07.02.2019 Gazette 2019/06)**

(54) **ENDOSCOPE SHAPE DISPLAY DEVICE, AND ENDOSCOPE SYSTEM**

ENDOSKOPFORMANZEIGEVORRICHTUNG UND ENDOSKOPSYSTEM

DISPOSITIF D'AFFICHAGE DE FORME D'ENDOSCOPE ET SYSTÈME D'ENDOSCOPE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.08.2017 JP 2017151729**

(43) Date of publication of application:
**12.02.2020 Bulletin 2020/07**

(73) Proprietor: **Hoya Corporation**
**Tokyo 160-8347 (JP)**

(72) Inventors:
• **ENOMOTO Takayuki**
**Tokyo 160-8347 (JP)**

• **NAKAYAMA Nobuhito**
**Tokyo 160-8347 (JP)**
• **NANCOLLIS, Peter Pearson**
**Portskewett**
**Caldicot NP26 5PS (GB)**

(74) Representative: **Schaumburg und Partner Patentanwälte mbB**
**Mauerkircherstraße 31**
**81679 München (DE)**

(56) References cited:
**WO-A1-2009/031456    WO-A1-2017/081821**
**JP-A- 2001 046 318    JP-A- 2002 325 721**
**JP-A- 2003 245 242    US-A- 6 059 718**
**US-A1- 2009 175 518**

EP 3 607 870 B1

**Description**

Technical Field

The present invention relates to a technique for displaying a shape of an endoscope.

Background Art

An endoscope is a device that observes inside of an observation object by inserting the endoscope into the observation object (for example, a human respiratory organ, digestive organ, and the like). For example, an endoscope can be used to observe the human large intestine. However, a certain level of skill is needed to accurately operate the endoscope in an observation object having a complicated shape. Therefore, in order to assist the operation of the endoscope, a technique is used in which a shape of a flexible portion of the endoscope inside the observation object is detected and displayed on a screen.

Patent Literature 1 below describes a technique for detecting a shape of an endoscope. The document discloses the technique to "provide an endoscope shape detection device by which an operator easily confirms whether the position is deviated from a detectable range with a specified accuracy". And "The detection device 21 for sensing the inserting shape of the endoscope is provided with a connector receiver 21a, etc., which the connector 16a, etc., of a probe 15 to be arranged at the insertion part of the endoscope is connected to or disconnected from. A CPU 32 detects connection, judges whether the endoscope is within an effective detection range where the position can be detected by a prescribed precision or precision higher than this based on detection output by sense coils 22a, 22b, etc., for detecting a magnetic field by a source coil 14i. Based on result of judgment, displaying color of a monitor 25 is changed to enable the operator to easily confirm whether the position is deviated from an effectively detectable range" (see the abstract).

Citation List

Patent Literature

Patent Literature 1: JP 2002-325721 A

Summary of Invention

Technical Problem

In the technique described in Patent Literature 1, when the entire endoscope is within the effective detection range, the shape is displayed as a green image, and when there is a portion of the endoscope outside the effective detection range, that portion is displayed in another color (for example, yellow) (see 0045 of the same document). The operator can confirm whether or not the endoscope is in the effective detection range based on the different display colors.

Although the technique described in Patent Literature 1 can be used to recognize on the screen whether or not the endoscope is included in the effective detection range; however, it is difficult to determine how reliable the shape of the endoscope displayed on the screen is.

The present invention has been made in view of the above described problem and provides an endoscope shape display device that is capable of providing an operator with information how reliable the provided shape of the endoscope is.

Solution to Problem

An endoscope shape display device according to the present invention calculates reliability of a result of estimating an endoscope shape, and displays, in a fade-out mode, at least a part of an image of the endoscope shape in conjunction with the reliability.

Advantageous Effects of Invention

The endoscope shape display device according to the present invention can provide an endoscope operator with information how reliable the shape of the endoscope, which is displayed on a screen, on the same screen.

Brief Description of Drawings

Fig. 1 is a configuration diagram of an endoscope system 1000 according to a first embodiment.
Fig. 2 is an internal configuration diagram of a CPU 230.
Fig. 3 is a schematic diagram illustrating changes with time in a shape of an endoscope 100 displayed on a monitor 300.
Fig. 4 is a flowchart for explaining an operation in which a processor 200 displays the shape of the endoscope 100 on a screen.
Fig. 5 is a flowchart for explaining a procedure by which a reliability calculation unit 233 calculates reliability of a shape estimation result.
Fig. 6 illustrates an example in which a part whose reliability has been recovered is displayed in a fade-in mode.
Fig. 7 illustrates an example in which fade-out and fade-in display is reversibly repeated according to an increase or a decrease in reliability.
Fig. 8 illustrates an example in which parameters that are basis for calculating the reliability are displayed together with the shape of the endoscope 100.
Fig. 9 illustrates an example in which a time change rate of the reliability is displayed together with the shape of the endoscope 100.
Fig. 10 is a schematic diagram illustrating changes with time in the shape of the endoscope 100 displayed on the monitor 300.
Fig. 11 is a flowchart illustrating an operation in which the processor 200 displays the shape of the endoscope 100 on the screen.
Fig. 12 is a flowchart for explaining a procedure by which the reliability calculation unit 233 calculates the reliability of the shape estimation result.
Fig. 13 illustrates an example of an image displayed on the screen by an endoscope system 1000 according to a fourth embodiment.
Fig. 14 is a configuration diagram of an endoscope system 1000 according to a fifth embodiment.
Fig. 15 is a diagram illustrating a configuration and example data of history data 241.
Fig. 16 illustrates an example of displaying a mark indicating that the reliability has decreased.
Fig. 17 illustrates an example of blinking a part having reduced reliability.
Fig. 18 illustrates an example of displaying a numerical value of the reliability.
Fig. 19 is an example of displaying a scale bar representing the reliability.

Description of Embodiments

<First Embodiment>

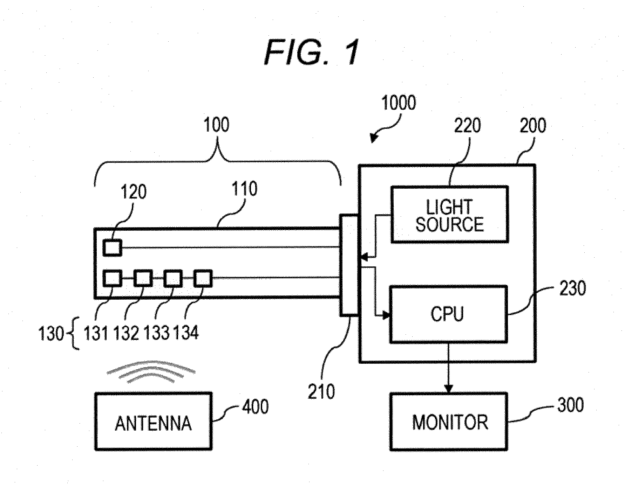

Fig. 1 is a configuration diagram of an endoscope system 1000 according to a first embodiment of the present invention. The endoscope system 1000 includes an endoscope 100, a processor 200 (an endoscope shape display device), a monitor 300, and an antenna 400.

The endoscope 100 is a device that is inserted into an observation object and observes the inside of the observation object. The endoscope 100 includes a flexible tube 110, an imaging device 120, and a sensor 130. The sensor 130 further includes a plurality of shape sensors (shape sensors 131 to 134 in Fig. 1).

The flexible tube 110 is a tube that is inserted into the observation object, and can be freely deformed to some extent according to the shape of the observation target. The imaging device 120 is provided at a distal end of the flexible tube 110, captures a peripheral image, and outputs an image signal. The sensor 130 is a sensor that detects a position and an orientation of the flexible tube 110.

The processor 200 is a device that processes a signal received from the endoscope 100. The processor 200 includes a receptacle 210, a light source 220, and a central processing unit (CPU) 230.

The receptacle 210 is an interface that connects the endoscope 100 and the processor 200. The light source 220 supplies light to the endoscope 100. The imaging device 120 can irradiate the observation object with light supplied from the light source 220 and capture an image of the irradiation region. The CPU 230 receives the image signal of the observation object from the imaging device 120 and displays the image on the screen of the monitor 300.

The antenna 400 generates a magnetic field, and the sensor 130 detects the magnetic field. The sensor 130 is configured by, for example, winding a coil around an iron core, and generates and outputs a current according to the magnetic field generated by the antenna 400. The processor 200 can calculate a position and an orientation of the endoscope 100 (more specifically, a position and an orientation of a part where the shape sensors 131 to 134 are arranged) using the current.

Since the method for calculating the position and orientation of the endoscope 100 is already known, detailed description thereof is omitted here. For example, by obtaining distance between each shape sensor and the antenna 400, the position of each shape sensor can be specified. For example, a posture of the endoscope 100 (the orientation of each part of the endoscope 100 in a three-dimensional coordinate system) can be specified by obtaining a three-dimensional curve connecting the positions of the shape sensors 131 to 134. Therefore, the sensor 130 serves as a position sensor of the endoscope 100 and as an angle sensor (a direction sensor) of the endoscope 100.

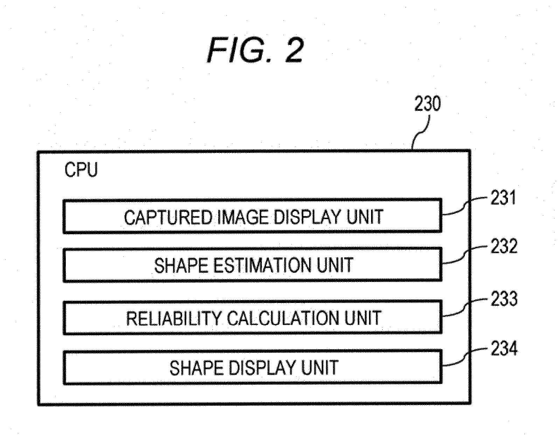

Fig. 2 is an internal configuration diagram of the CPU 230. The CPU 230 includes a captured image display unit 231, a shape estimation unit 232, a reliability calculation unit 233, and a shape display unit 234. These functional units may be composed of hardware such as a circuit device, or the functional units may be configured by installing software and executing the software by the CPU 230. In the following, for convenience of description, each functional unit will be described as an operating entity, but the actual operating entity is the CPU 230 in a case where these functional units are installed as software. Each software can be stored in an appropriate storage device such as a memory included in the CPU 230.

The captured image display unit 231 generates an image of an observation object by processing the image signal output from the imaging device 120 and displays the image on the monitor 300. The shape estimation unit 232 estimates the shape (the position and orientation) of the endoscope 100 using a signal output from the sensor 130. The reliability calculation unit 233 calculates the reliability of the shape of the endoscope 100 estimated by the shape estimation unit 232 by a later described method. The shape display unit 234 displays an image of the shape of the endoscope 100 estimated by the shape estimation unit 232 on the monitor 300.

The accuracy of the result that the shape estimation unit 232 estimates the shape of the endoscope 100 is considered to be constantly changing. For example, when distance between any of the shape sensors 131 to 134 and the antenna 400 exceeds a detectable range, it is assumed that the signal output by the shape sensor has low accuracy. Even when the sensor 130 is within the detectable range, for example, if there is a significant change in the position or shape, the detection accuracy itself by the sensor 130 decreases and this may also cause decrease of estimation accuracy.

When a shape estimation accuracy of a part of the endoscope 100 is decreased, displaying that the decrease of the estimation accuracy on the screen is useful for the operator of the endoscope 100. This is because the estimation accuracy may be increased by manipulating and changing the position and orientation of the part, for example. On the other hand, when the estimation accuracy is decreased, the operator may be confused if the display mode of the part is rapidly changed on the screen. This is because the shape of the endoscope 100 has normally been displayed on the screen until the time immediately before the change.

Therefore, according to the first embodiment of the present invention, the reliability calculation unit 233 calculates the reliability of the estimation result by the shape estimation unit 232, and the shape display unit 234 changes the screen display of the endoscope 100 over time according to the reliability. As a result, since the shape of the endoscope 100 on the screen also changes with time in accordance with the change in reliability over time, the operator may have less confusion caused by sudden screen changes.

The reliability calculation unit 233 calculates the reliability of the shape of the endoscope 100 estimated by the shape estimation unit 232 using the following Equation 1 or 2. Each parameter of each equation will be described below.

$$\text{reliability} = \text{sensor accuracy} \times (1/\text{shape change degree}) \ (\text{Equation 1})$$

$$\text{reliability} = \text{sensor accuracy} \times (1/\text{shape change degree}) \times (1/\text{elapsed time}) \ (\text{Equation 2})$$

The sensor accuracy is a value representing detection accuracy by the sensor 130, and can be expressed by a numerical value between 0.0 and 1.0, for example. Since intensity of current output from the sensor 130 corresponds to the distance between the sensor 130 and the antenna 400 for example, a higher current intensity can be considered to indicate a higher detection accuracy. As an example, when the output of the sensor 130 is an upper limit of a rated value, the sensor accuracy can be regarded as 1.0, and when no signal is obtained, the sensor accuracy can be regarded as 0.0. Since the sensor 130 serves as both the position sensor and angle sensor, the sensor accuracy represents both the position accuracy and angle accuracy.

If any of the shape sensors 131 to 134 is broken, it is considered that the reliability of the broken shape sensor is the lowest. Note that, in a case where the detection result of the broken shape sensor can be complemented with a detection result of another shape sensor, the reliability of the broken shape sensor can be improved according to the reliability of the complement. For example, the reliability of the failed shape sensor can be complemented by (a) averaging the detection values of other shape sensors, (b) multiplying the reliability of other shape sensors with an appropriate weight (a greater weight is applied to the sensor closer to the broken shape sensor, for example), and the like.

The shape change degree is a value representing a rate of change of the position of the endoscope 100 with time. When the position of the endoscope 100 is changing at high speed, the detection accuracy by the sensor 130 is considered to be low and the estimation accuracy can also be considered low. For example, the moving speed (or an equivalent numerical value) of each shape sensor can be used as the shape change degree.

Even when the position of the endoscope 100 is changing at high speed, if the posture of the endoscope 100 (the orientation of each shape sensor) is kept constant, the estimation accuracy is considered to be higher compared to a case where both the posture and the position are changing. This is because if the posture of the endoscope 100 is constant, the position can be estimated with a certain degree of accuracy by interpolation calculation or the like. In view of the above, the reliability calculation unit 233 can also use the change rate of the posture of the endoscope 100 with respect to the time, in addition to the position change rate. The posture change rate can be obtained, for example, as the time change rate of the orientation of each shape sensor. For example, the shape change degree can be obtained by multiplying the position change rate and the posture change rate. In addition, the position change rate and the posture change rate may be combined by an appropriate calculation formula.

Elapsed time is time passed from the time when the reliability is lower than a predetermined threshold value. The reliability calculation unit 233 basically calculates the reliability using Equation 1; however, when the reliability is lower than a predetermined threshold value, a final reliability is determined by further multiplying the reliability with an inverse number of the subsequent elapsed time according to Equation 2.

When the reliability decreases below a certain level, it is considered that the reliability further decreases as time passes. This is because it is not desirable to keep the state where the shape of the endoscope 100 is unreliable for a long time. In view of the above, the reliability calculation unit 233 is configured to multiply the reliability with the inverse number of the elapsed time. Note that, in a case where the multiplication of the sensor accuracy with the shape change degree becomes lower than the predetermined threshold and then recovers to be equal to or greater than the predetermined threshold value, the elapsed time is reset, and thereafter, Equation 1 is used as the reliability (the inverse number of the elapsed time is not multiplied). The reliability calculation unit 233 repeats the above process and constantly updates the reliability.

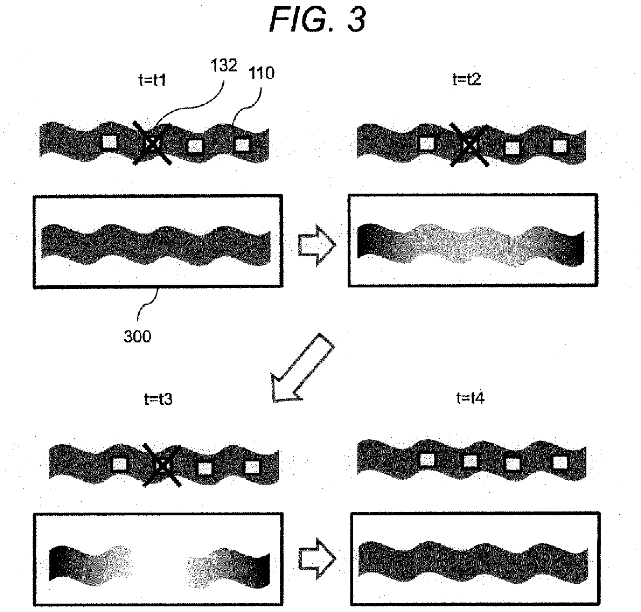

Fig. 3 is a schematic diagram illustrating a change of the shape of the endoscope 100 over time, which is displayed on the monitor 300. It is assumed that, at time t = t1, the detection accuracy of the shape sensor 132 decreases, and the estimation reliability of the part corresponding to the shape sensor 132 is lower than the threshold value. The reliability calculation unit 233 calculates the reliability by multiplying the inverse number of the time elapsed from the time t1 according to Equation 2. As a result, the reliability of the part corresponding to the shape sensor 132 decreases as the time elapses as t1 => t2 => t3.

The shape display unit 234 displays a portion of the endoscope 100 in which the reliability becomes lower than the threshold value in a fade-out manner according to the reliability value. In Fig. 3, as time elapses as t1 => t2 => t3, the part corresponding to the shape sensor 132 fades out as the reliability of the part decreases. The fade out here means that the image gradually disappears. The image does no need to completely disappear, and a change in display mode in the process of completely disappearing is also included in the fade-out here.

It is assumed that the detection accuracy of the shape sensor 132 has recovered to a normal level at time t4. As a result, the multiplication of the sensor accuracy and the shape change degree recovers equal to or greater than the threshold value. The reliability calculation unit 233 calculates the reliability according to Equation 1 after time t4. Since the reliability of the part corresponding to the shape sensor 132 has recovered equal to or greater than the threshold value, the shape display unit 234 returns the display of the part to the normal mode.

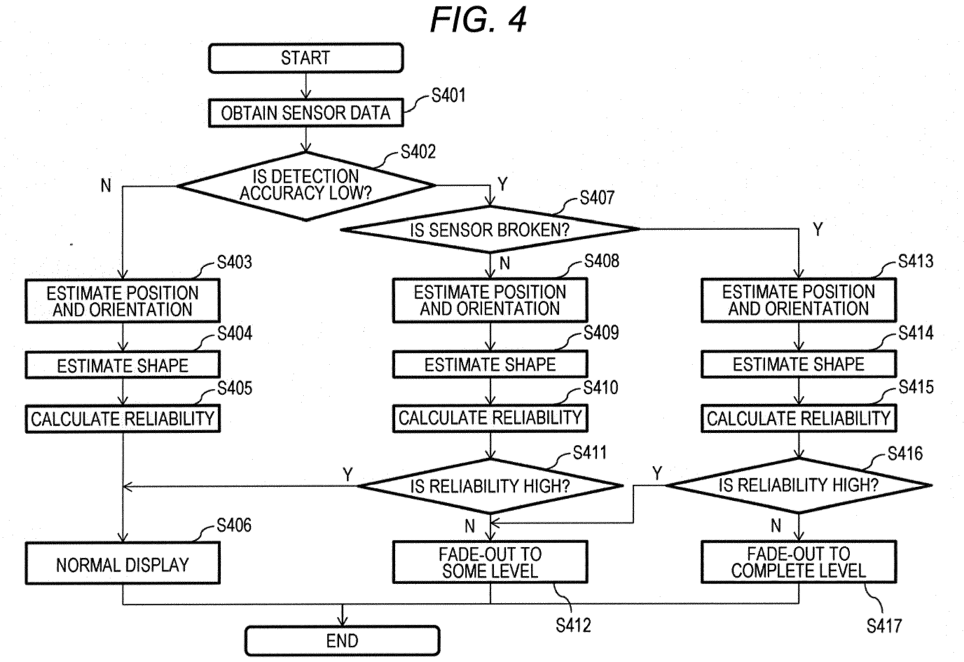

Fig. 4 is a flowchart for explaining operation in which the processor 200 displays the shape of the endoscope 100 on the screen. The processor 200 repeats executing this flowchart and displays the shape of the endoscope 100 on the screen of the monitor 300. In the following, each step of Fig. 4 will be described.

(Fig. 4: Step S401)

The shape estimation unit 232 acquires a detection value from the sensor 130. At this time, data representing the detection accuracy of the sensor 130 is also acquired. In a case where the sensor 130 itself outputs a value representing the detection accuracy, the output is acquired. Alternatively, for example, a value such as an intensity of the current output from the sensor 130 can be used as an index of detection accuracy. The detection accuracy may be acquired by other appropriate methods.

(Fig. 4: Step S402)

The shape estimation unit 232 determines whether or not the detection accuracy of the sensor 130 is lower than a detection accuracy threshold value. When the accuracy is lower than the threshold value, the process proceeds to step S407, and when the accuracy is equal to or greater than the detection accuracy threshold value, the process proceeds to step S403.

(Fig. 4: Steps S403 to S405)

The shape estimation unit 232 estimates the position and orientation of each part of the endoscope 100 based on the detection value obtained from the sensor 130 (S403). The shape estimation unit 232 estimates the shape of the endoscope 100 based on the estimation result in step S403 (S404). The reliability calculation unit 233 calculates the reliability of the estimation result in step S404 using Equation 1 (S405).

(Fig. 4: Step S405: Supplementary explanation)

When the detection accuracy of the sensor 130 is sufficiently high, the accuracy of the shape estimation result of the endoscope 100 is also assumed to be high. Therefore, in order to simplify the processing, instead of using Equation 1 in this step, a sufficiently high fixed value (for example, maximum value = 1.0) can be assigned as the reliability value.

(Fig. 4: Step S406)

The shape display unit 234 displays the estimation result by the shape estimation unit 232 on the screen of the monitor 300. In this step, since the reliability of the estimation result by the shape estimation unit 232 is considered to be sufficiently high, the fade-out display as illustrated in Fig. 3 is not performed.

(Fig. 4: Step S406: Supplementary explanation)

When the process reaches this step, the reliability is considered sufficiently high and the fade-out display is not necessary. However, if there is a possibility that the reliability is lower than the reliability threshold value in this step, the reliability calculation unit 233 and the shape display unit 234 decrease the reliability over time as in later described step S412 and the shape of the endoscope 100 may be displayed in a faded-out mode.

(Fig. 4: Step S407)

The shape estimation unit 232 determines whether or not the sensor 130 is broken. When the sensor 130 itself outputs a signal indicating the presence or absence of a failure, the determination may be made based on the signal. Alternatively, the presence or absence of a failure may be determined based on a state where the sensor 130 does not output any detection value, or the like. The presence or absence of a failure may be determined by any other appropriate method. In a case where the sensor 130 is broken, the process proceeds to step S413, and in a case where the sensor 130 is not broken (the detection accuracy has simply decreased), the process proceeds to step S408.

(Fig. 4: Step S407: Supplementary explanation)

A state in which the sensor 130 is not broken but the detection accuracy is lowered may be, for example, a case where the strength of the signal output from the sensor 130 is lowered due to an increased distance between the sensor 130 and the antenna 400. In addition, since there is a piece of metal in the vicinity of the antenna 400 and the sensor 130 may reduce the detection accuracy.

(Fig. 4: Steps S408 to S410)

The shape estimation unit 232 and the reliability calculation unit 233 perform similar processing as in steps S403 to S405.

(Fig. 4: Step S411)

The reliability calculation unit 233 determines whether or not the reliability calculated in step S410 is lower than a reliability threshold value. In a case where the reliability is lower than the reliability threshold value, the process proceeds to step S412 and, in a case where the reliability is equal to or greater than the reliability threshold value, the process proceeds to step S406.

(Fig. 4: Step S412)

The reliability calculation unit 233 lowers the reliability of the part where the detection accuracy of the sensor 130 is lower than the detection accuracy threshold value over time not to let the reliability be zero. For example, the reliability is decreased over time, and the reliability value is fixed when the reliability is lowered to a certain extent. The shape display unit 234 displays the shape of the endoscope 100 on the screen according to the changes in reliability over time. With this configuration, the shape of the endoscope 100 fades out to the extent that the shape does not completely disappear. For example, the shape is displayed in the display mode of t = t2 in Fig. 3.

(Fig. 4: Step S412: Supplementary explanation)

In this step, as in step S417, the shape of the endoscope 100 may be faded out until the shape of the endoscope 100 completely disappears. In this case, in order to visually display the difference between steps S412 and S417, the reliability needs to be displayed on the screen in a mode other than the fade-out mode as in a later described second embodiment.

(Fig. 4: Steps S413 to S415)

The shape estimation unit 232 and the reliability calculation unit 233 perform similar processing as in steps S403 to S405.

(Fig. 4: Step S416)

The reliability calculation unit 233 determines whether or not the reliability calculated in step S415 is lower than the reliability threshold value. In a case where the reliability is lower than the reliability threshold value, the process proceeds to step S417 and, in a case where the reliability is equal to or greater than the reliability threshold value, the process proceeds to step S412.

(Fig. 4: Step S417)

The reliability calculation unit 233 lowers the reliability of the part where the detection accuracy of the sensor 130 is lower than the detection accuracy threshold value to be zero with time. The shape display unit 234 displays the shape of the endoscope 100 on the screen according to the changes in reliability over time. With this configuration, the shape of the endoscope 100 fades out until the shape completely disappears. For example, the shape is displayed in the display mode of t = t3 in Fig. 3.

(Fig. 4: Steps S412 and S417 Supplementary explanation 1)

The difference between steps S412 and S417 depends on whether or not the sensor 130 is broken. In a case where the sensor 130 is broken, the reliability of the shape estimation result of the part is considered to be low, and this helps to visually suggest the operator that the reliability is low by completely erasing the part on the monitor 300. On the other hand, in step S412, since the detection accuracy of the sensor 130 is considered to be temporarily lowered, the operator is visually suggested that the condition is different from that in S417 by keeping the same part on the monitor 300 without completely erasing the shape. With this configuration, the reliability of the shape estimation result can be visually suggested by the image on the monitor 300, so that the operator can be effectively assisted.

(Fig. 4: Steps S412 and S417 Supplementary explanation 2)

There may be a case that the reliability of the shape estimation result may recover to be equal to or greater than the reliability threshold value by executing this flowchart again after the fade-out display is started in this step. In such a case, the fade-out display in this step can be stopped and the display returns to the normal display. For example, in step S406, the display mode may be returned to the normal display.

(Fig. 4: steps S406 and S412 Supplement explanation)

Even when the detection accuracy of the sensor 130 is temporarily lowered, the display does not need to be faded out if the shape estimation result is sufficiently reliable. Therefore, when the reliability is sufficiently high in step S411, the same process (S406) as in a case of a high detection accuracy is performed. On the other hand, for example, when the shape change degree of the endoscope 100 is large, the reliability is calculated to be low by Equation 1 and thus the process in step S412 is performed.

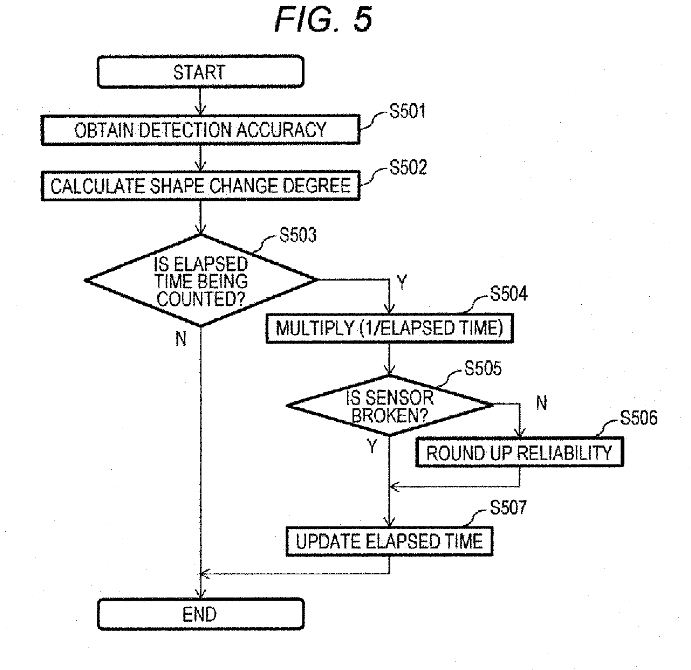

Fig. 5 is a flowchart for explaining a procedure by which the reliability calculation unit 233 calculates the reliability of the shape estimation result. This flowchart is performed when the reliability calculation unit 233 calculates the reliability in Fig. 4. Hereinafter, each step of Fig. 5 will be described.

(Fig. 5: Step S501)

The reliability calculation unit 233 acquires the detection accuracy of the sensor 130. More specifically, the detection accuracy acquired from the sensor 130 by the shape estimation unit 232 in step S401 may be received from the shape estimation unit 232.

(Fig. 5: Step S502)

The reliability calculation unit 233 calculates the shape change degree of the endoscope 100. The shape change degree can be calculated by using one or more of the following parameters, for example. (a) Time change rate of the position of the endoscope 100 (the position detected by the sensor 130), (b) Time change rate of a tangent vector of the shape of the endoscope 100, (c) Time change rate of the posture of the endoscope 100 (the orientation detected by the sensor 130), and (d) Radius of curvature of a local shape of the endoscope 100 The reliability calculation unit 233 calculates the reliability using Equation 1.

(Fig. 5: Step S503)

The reliability calculation unit 233 determines whether or not the elapsed time since the reliability become lower than the reliability threshold value is being counted. In a case where this flowchart is executed in step S405, S410, or S415, since the elapsed time is not counted, this flowchart is ended. In a case where this flowchart is executed in step S406, S412, or S417, since the shape of the endoscope 100 is displayed in a fade-out manner, step S504 and subsequent steps are executed.

(Fig. 5: Step S504)

The reliability calculation unit 233 multiplies the reliability calculated in step S502 by (1/elapsed time). The elapsed time here is the elapsed time from the time when the reliability is determined to be lower than the reliability threshold value in step S411 or S416.

(Fig. 5: Step S505)

The reliability calculation unit 233 determines whether or not the sensor 130 is broken by the similar procedure as in step S407. In a case where the sensor 130 is broken, the process skips to step S507 and, in a case where the sensor 130 is not broken, the process proceeds to step S506. Step S507 corresponds to step S417, and step S506 corresponds to step S412.

(Fig. 5: Step S506)

The reliability calculation unit 233 rounds up to the lower limit value when the reliability is lower than the lower limit value. This step is to keep the shape of the endoscope 100 not to be completely erased after displaying in the fade-out mode.

(Fig. 5: Step S507)

The reliability calculation unit 233 updates the elapsed time. The elapsed time can be measured by using, for example, a timer that starts counting up in step S411 or S416.

<First Embodiment: Summary>

The endoscope system 1000 according to the first embodiment performs fade-out display of the shape of the endoscope 100 on the monitor 300 in conjunction with the reliability of the shape of the endoscope 100 estimated by the shape estimation unit 232. With this configuration, regarding the shape estimation result of the endoscope 100, visual feedback is given to the operator to assist the operator. In particular, the result of estimating the shape of the endoscope does not necessarily have 100% accuracy, and since the reliability of the estimation result constantly changes, recognizing the shape using the endoscope system 1000 according to the first embodiment is useful. In particular, it is preferable for displaying the insertion shape of the endoscope.

As described in Patent Literature 1, when one threshold value is provided and displayed as changing colors such as red and yellow, there is a problem that a sudden change in display or flickering occurs and this causes a problem that the usability is lowered. In view of this problem, the endoscope system 1000 according to the first embodiment does not perform binary display, but the fade-out display according to reliability, or determines the reliability of a missing sensor (a sensor that the processor 200 can no longer receive a signal) based on adjacent sensors to maintain continuity of the reliability. This configuration can solve the above-mentioned problem.

<Second Embodiment>

In a second embodiment of the present invention, various display modes for a case where the shape display unit 234 displays the shape of the endoscope 100 on the screen of the monitor 300 will be described. The configuration of the endoscope system 1000 is similar to that of the first embodiment.

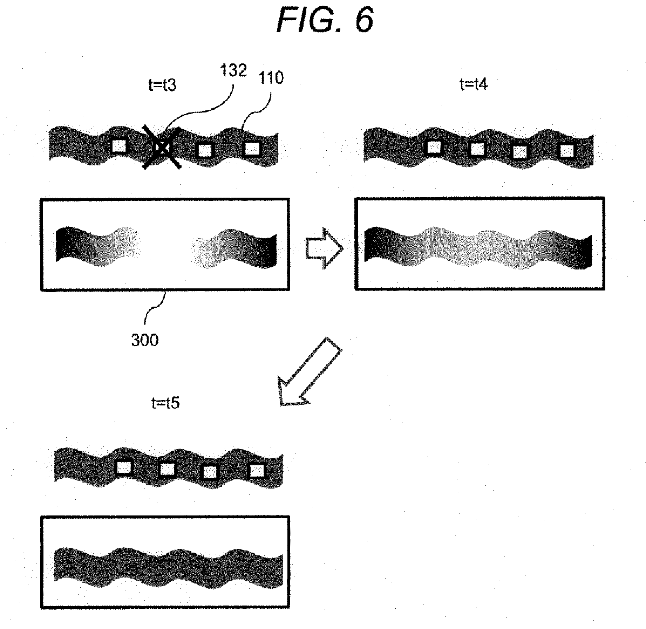

Fig. 6 illustrates an example in which a part whose reliability has been recovered is displayed in a fade-in mode. In Fig. 3, the shape display unit 234 immediately returns the shape of the corresponding part to the normal mode when the reliability is recovered to be equal to or greater than a predetermined threshold value. Instead of the above, the shape display unit 234 may perform fade-in display of an image of a part whose reliability has been recovered to be equal to or greater than the reliability threshold value. The time interval of the fade-in process may be uniform or variable depending on the speed of recovery of reliability.

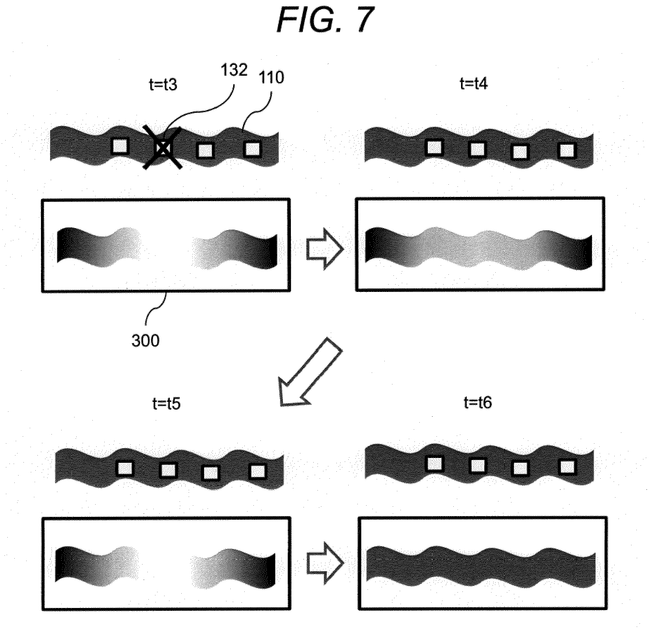

Fig. 7 illustrates an example in which the fade-out and fade-in modes are reversibly repeated in accordance with increase or decrease in reliability. In the first embodiment, the reliability calculation unit 233 switches from Equation 1 to Equation 2 to perform fade-out display when the reliability is lower than the reliability threshold value. Instead of the above, the reliability calculation unit 233 may continue to calculate the reliability using Equation 1 even after the reliability becomes lower than the reliability threshold value. In this case, the reliability value does not necessarily decrease in a monotonous manner, and may increase and decrease repeatedly. The shape display unit 234 repeats the fade-out and fade-in modes of the image of the endoscope 100 as the reliability increases or decreases.

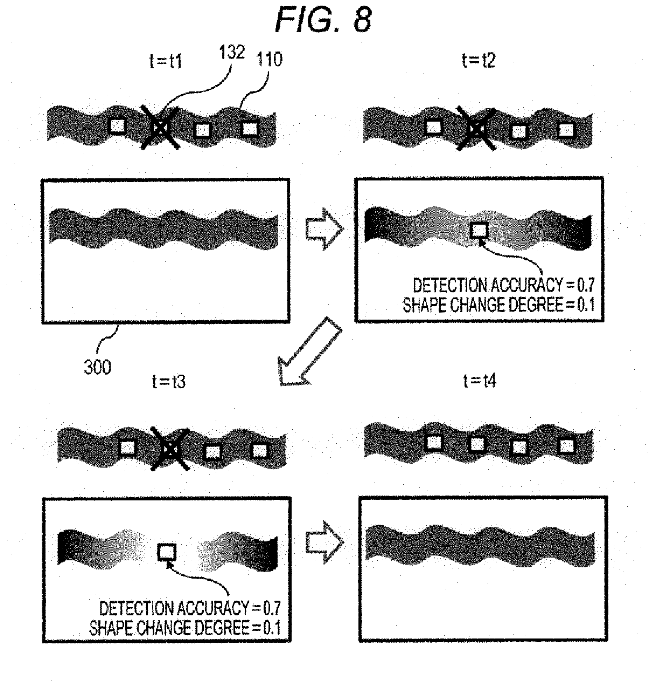

Fig. 8 is an example in which a parameter that is a basis for calculating the reliability is displayed together with the shape of the endoscope 100. By displaying the part with reduced reliability in a fade-out mode, the part is suggested to the operator; however, the operator can recognize its reason when the reason why the reliability has decreased to the operator in addition to the above. In other words, this prevents the operator from being anxious due to the decrease in reliability for unknown reasons. The reason of the decrease in reliability may be at least two parameters in Equation 1. In addition, the position accuracy and the angle accuracy may be individually displayed. In Fig. 8, the parameters are displayed by character strings; however, equivalent information can also be presented by appropriate symbols, icon images, and the like.

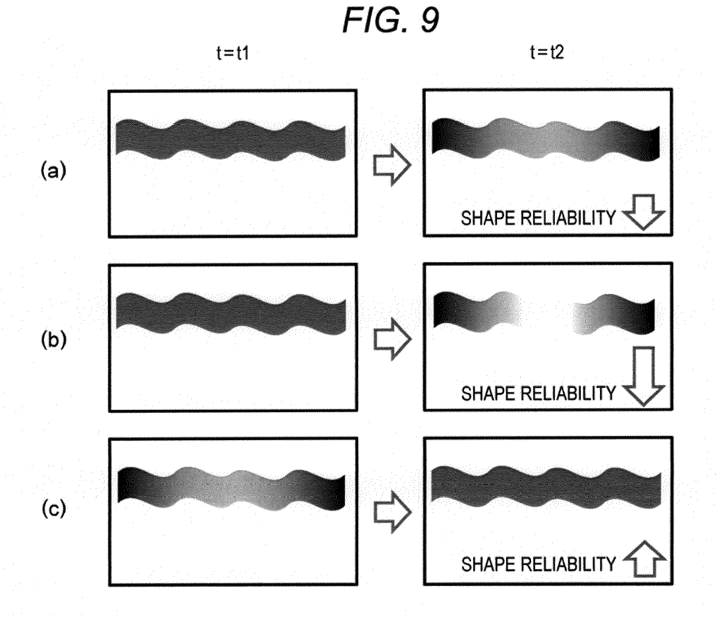

Fig. 9 is an example in which the time change rate of the reliability is displayed together with the shape of the endoscope 100. In the example of Fig. 9(a), since the reliability is gradually decreased, a short downward arrow is displayed on the screen. In the example of Fig. 9(b), since the reliability decreases sharply, a long arrow is displayed on the screen. In the example of Fig. 9(c), since the reliability increases moderately, a short upward arrow is displayed on the screen. Appropriate notation other than the arrows may be used. By displaying the time change rate of the reliability, the operator can visually recognize on the screen whether or not the endoscope 100 is appropriately operated.

The display modes described in Figs. 6 to 9 can be used in any combination. For example, the display mode for repeating the fade-in and fade-out modes described in Fig. 7 and the icon indicating the reliability change rate described in Fig. 9 can be used in combination.

<Third Embodiment>

In the third embodiment of the present invention, a display mode other than the fade-out and fade-in modes to display the shape image of the endoscope 100 will be described. The configuration of the endoscope system 1000 is similar to those in the first and second embodiments.

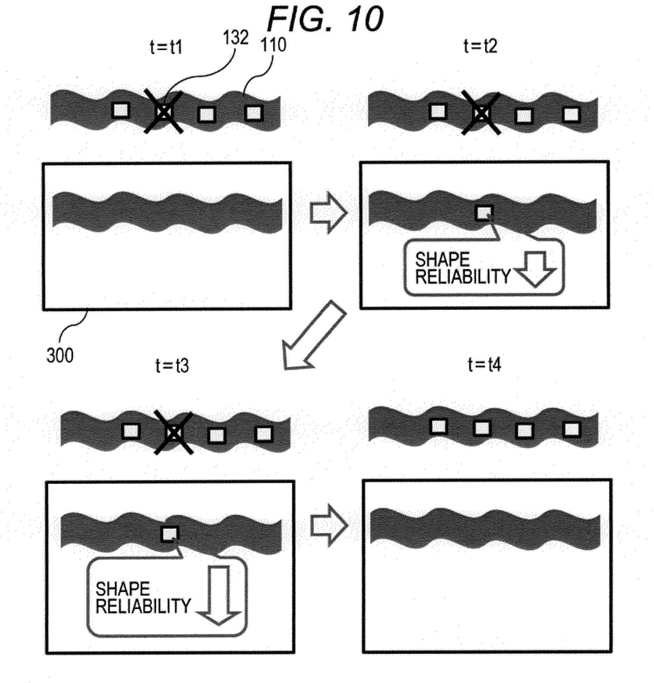

Fig. 10 is a schematic diagram illustrating changes with time in the shape of the endoscope 100 displayed on the monitor 300. It is assumed that, at time t = t1, the detection accuracy of the shape sensor 132 decreases, and the estimation reliability of the part corresponding to the shape sensor 132 is lower than the threshold value. The reliability calculation unit 233 calculates the reliability by multiplying the inverse number of the time elapsed from the time t1 according to Equation 2. As a result, the reliability of the part corresponding to the shape sensor 132 decreases as the time elapses as t1 => t2 => t3.

The shape display unit 234 displays an image indicating reduction of the reliability in a position of the vicinity of the part of the endoscope 100 where the reliability has decreased to be lower than the threshold value or in a position corresponding to the relevant part. In Fig. 10, as the time elapses as t1 => t2 => t3, the reliability of the part corresponding to the shape sensor 132 gradually decreases. The shape display unit 234 displays, on the screen, a downward arrow indicating that the reliability is lowered in a pop-up indicating the part corresponding to the shape sensor 132. The length of the arrow represents how much the reliability is reduced. In Fig. 10, since the arrow becomes longer as time elapses, it is visually suggested that the reliability is gradually decreasing.

It is assumed that the detection accuracy of the shape sensor 132 has recovered to a normal level at time t4. As a result, the multiplication of the sensor accuracy and the shape change degree recovers equal to or greater than the threshold value. The reliability calculation unit 233 calculates the reliability according to Equation 1 after time t4. Since the reliability of the part corresponding to the shape sensor 132 has recovered equal to or greater than the threshold value, the shape display unit 234 returns the display of the part to the normal mode.

The shape display unit 234 may display so that the length of the arrow itself indicates the reliability, or may display so that the change rate of the reliability is expressed by the length and direction of the arrow. For example, a long downward arrow can be displayed when the reliability sharply decreases, and a short upward arrow can be displayed when the reliability increases moderately.

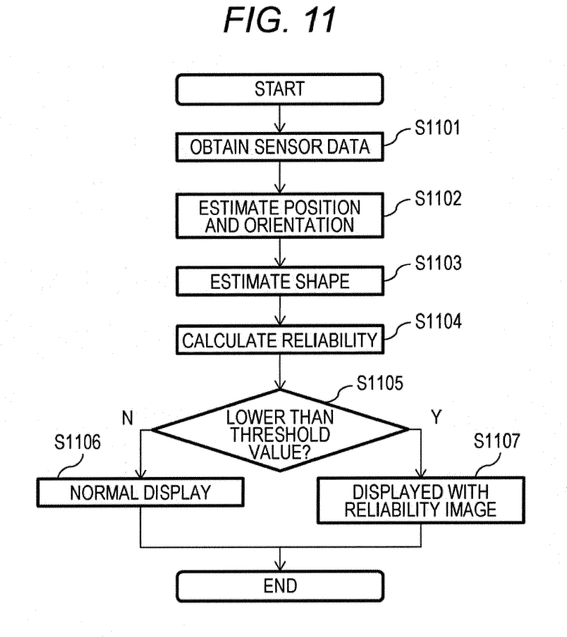

Fig. 11 is a flowchart for explaining operation in which the processor 200 displays the shape of the endoscope 100 on the screen. The processor 200 repeats executing this flowchart and displays the shape of the endoscope 100 on the screen of the monitor 300. Hereinafter, each step of Fig. 11 will be described.

(Fig. 11: Step S1101)

The shape estimation unit 232 acquires a detection value from the sensor 130. At this time, data representing the detection accuracy of the sensor 130 is also acquired. In a case where the sensor 130 itself outputs a value representing the detection accuracy, the output is acquired. Alternatively, for example, a value such as an intensity of the current output from the sensor 130 can be used as an index of detection accuracy. The detection accuracy may be acquired by other appropriate methods.

(Fig. 11: Steps S1102 to S1104)

The shape estimation unit 232 estimates the position and orientation of each part of the endoscope 100 based on the detection value acquired from the sensor 130 (S1102). The shape estimation unit 232 estimates the shape of the endoscope 100 based on the estimation result of step S1102 (S1103). The reliability calculation unit 233 calculates the reliability of the estimation result in step S1104 according to Equation 1 (S1104).

(Fig. 11: Step S1105)

The shape display unit 234 determines whether or not the reliability calculated by the reliability calculation unit 233 in step S1104 is lower than the reliability threshold value. In a case where the reliability is lower than the threshold value, the process proceeds to step S1107, and in a case where the reliability is equal to or greater than the threshold value, the process proceeds to step S1106.

(Fig. 11: Step S1106)

The shape display unit 234 displays the estimation result by the shape estimation unit 232 on the screen of the monitor 300. In this step, since the reliability of the estimation result by the shape estimation unit 232 is sufficiently high, the reliability image as illustrated in Fig. 10 is not displayed.

(Fig. 11: Step S1107)

The reliability calculation unit 233 decreases the reliability over time for a part where the detection accuracy of the sensor 130 is lower than the detection accuracy threshold value. The shape display unit 234 displays a reliability image representing the reliability calculated by the reliability calculation unit 233 on the screen together with the estimation result by the shape estimation unit 232. For example, the display is in the display mode as illustrated in t = t2 or t = t3 in Fig. 10.

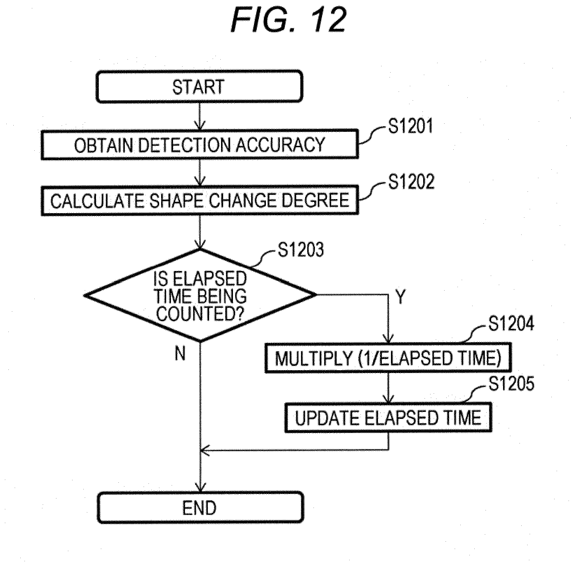

Fig. 12 is a flowchart for explaining a procedure by which the reliability calculation unit 233 calculates the reliability of the shape estimation result. This flowchart is performed when the reliability calculation unit 233 calculates the reliability in Fig. 11. Hereinafter, each step of Fig. 12 will be described.

(Fig. 12: Step S1201)

The reliability calculation unit 233 acquires the detection accuracy of the sensor 130. More specifically, the detection accuracy acquired from the sensor 130 by the shape estimation unit 232 in step S1101 may be received from the shape estimation unit 232.

(Fig. 12: Step S1202)

The reliability calculation unit 233 calculates the shape change degree of the endoscope 100. The shape change degree can be calculated by using one or more of the following parameters, for example. (a) Time change rate of the position of the endoscope 100 (the position detected by the sensor 130), (b) Time change rate of a tangent vector of the shape of the endoscope 100, (c) Time change rate of the posture of the endoscope 100 (the orientation detected by the sensor 130), and (d) Radius of curvature of a local shape of the endoscope 100 The reliability calculation unit 233 calculates the reliability using Equation 1.

(Fig. 12: Step S1203)

The reliability calculation unit 233 determines whether or not the elapsed time since the reliability become lower than the reliability threshold value is being counted. When this flowchart is executed in step S1106, the elapsed time is not counted, and thus this flowchart is ended. When this flowchart is executed in step S1107, the reliability is lowered according to the elapsed time, and therefore step S1204 and subsequent steps are executed.

(Fig. 12: Step S1204)

The reliability calculation unit 233 multiplies the reliability calculated in step S1202 by (1/elapsed time). The elapsed time here is the elapsed time from the time when it is determined in step S1105 that the reliability is lower than the reliability threshold value.

(Fig. 12: Step S1205)

The reliability calculation unit 233 updates the elapsed time. The elapsed time can be measured by using a timer or the like that starts counting up in step S1107, for example.

<Third Embodiment: Summary>

The endoscope system 1000 according to a third embodiment displays a reliability image representing the reliability of the estimation result together with the shape of the endoscope 100 estimated by the shape estimation unit 232. With this configuration, regarding the shape estimation result of the endoscope 100, visual feedback is given to the operator to assist the operator.

<Fourth Embodiment>

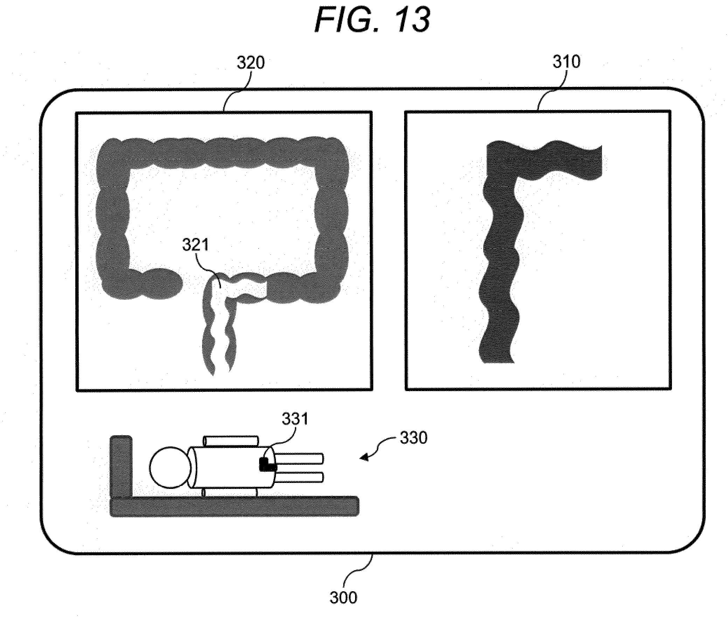

Fig. 13 is an example of an image displayed on the screen by the endoscope system 1000 according to a fourth embodiment of the present invention. In the fourth embodiment, the shape display unit 234 displays the image illustrated in Fig. 13 on the monitor 300 in addition to the image described in the third embodiment. Hereinafter, each image in Fig. 13 will be described.

A shape display field 310 is an area for displaying a shape image and reliability image of the endoscope 100. The shape display unit 234 displays the shape image and reliability image described in the first embodiment on the shape display field 310.

An internal structure field 320 is an area that displays an internal structure image that schematically illustrates an internal structure of the object into which the endoscope 100 is to be inserted. Fig. 13 illustrates an example in which a schematic image of the large intestine is displayed on an assumption that the endoscope 100 is inserted into the large intestine of the human body. A plurality of images (for example, large intestine, bronchi, and the like) are stored in advance on a storage device provided in the processor 200 as the internal structure image, and the operator designates any internal structure image via an appropriate interface. The processor 200 receives the designation, and the shape display unit 234 reads the corresponding internal structure image from the storage device and displays the image on the screen.

The shape display unit 234 further displays the shape image 321 on the screen on the internal structure field 320. The shape image 321 is an image representing the approximate position and shape of the endoscope 100 on the internal structure in which the endoscope 100 is inserted. Since the sensor 130 detects the position and orientation of the endoscope 100, the shape estimation unit 232 can use the detection to estimate the position and shape of the endoscope 100 on the internal structure. The shape display unit 234 displays the position and shape on the internal structure of the endoscope 100 estimated by the shape estimation unit 232 as a shape image 321 on the screen.

As a method for estimating the position and shape of the endoscope 100 on the internal structure, for example, the following methods can be considered although other appropriate methods may also be used: (a) A relative position and a relative posture of the human body with respect to the antenna 400 is detected by capturing the human body for example, and three-dimensional coordinates of the internal structure is estimated based on the result. By associating the three-dimensional coordinates of the internal structure with the three-dimensional coordinates of the endoscope 100, the position and shape of the endoscope 100 on the internal structure can be estimated. (b) When a distal end of the endoscope 100 reaches an entrance of the internal structure, the operator instructs the processor 200 regarding the condition. The shape estimation unit 232 estimates the position and shape of the endoscope 100 on the internal structure according to the subsequent movement of the endoscope 100.

The human body image display field 330 is a field for displaying a human body image that schematically represents the human body into which the endoscope 100 is inserted. The human body image can be stored in advance on the storage device included in the processor 200, for example. The shape estimation unit 232 estimates the position and posture of the human body by the method as described above (a), and modifies the human body image (adjusts the position and orientation) according to the estimation result. The shape display unit 234 displays the human body image on the screen.

The shape display unit 234 further displays a shape image 331 on the screen on the human body image display field 330. The shape image 331 is an image having the same role as the shape image 321. Since the position and orientation of the internal structure image inside the human body can be recognized in advance, the shape estimation unit 232 can estimate the position and shape of the endoscope 100 on the human body image when estimating the position and shape of the endoscope 100 on the internal structure. The shape display unit 234 displays the position and shape of the endoscope 100 on the human body image estimated by the shape estimation unit 232 as a shape image 331 on the screen.

As illustrated in Fig. 13, in a case where the position of the endoscope 100 on the internal structure to which the endoscope 100 is inserted is displayed on the screen together with the shape image of the endoscope 100, since the operator properly recognizes the position of the endoscope 100, more effective operation assistance information can be provided to the operator.

<Fifth Embodiment>

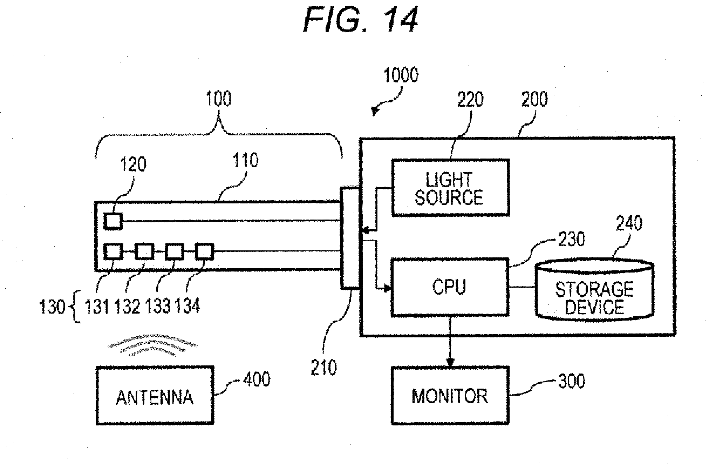

Fig. 14 is a configuration diagram of an endoscope system 1000 according to the fifth embodiment of the present invention. In the fifth embodiment, the processor 200 includes a storage device 240. The storage device 240 is a non-volatile storage device such as a hard disk drive for example. The storage device 240 stores later described history data 241 in addition to each image described in the second embodiment.

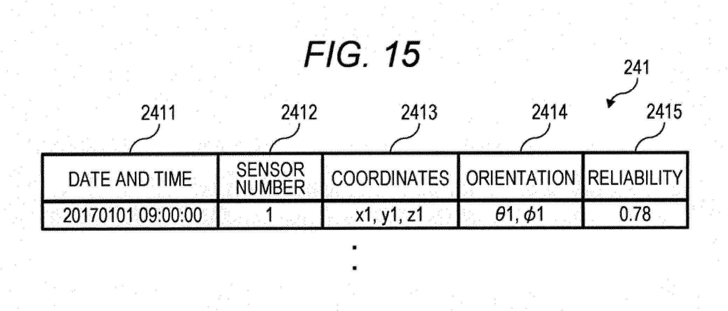

Fig. 15 is a diagram illustrating a configuration of the history data 241 and a data example. The history data 241 is a data table that records history of estimation results by the shape estimation unit 232 that estimates the shape of the endoscope 100. The history data 241 includes date and time 2411, sensor number 2412, coordinates 2413, orientation 2414, and reliability 2415 as data fields.

The date and time 2411 are the date and time when the shape estimation unit 232 estimates the shape of the endoscope 100. The sensor number 2412 is a number identifying one of the shape sensors 131 to 134. Coordinates 2413 are estimation results of the shape estimation unit 232 that estimates the coordinates of the sensor 130 based on the detection result of the sensor 130. The orientation 2414 is an estimation result of the shape estimation unit 232 that estimates the direction of the sensor 130 based on the detection result of the sensor 130. The reliability 2415 is a reliability value calculated by the reliability calculation unit 233.

The history data 241 can be used, for example, to analyze a reason of the decrease in reliability. The reason of the decrease of the reliability may be that the sensor 130 is separated from the antenna 400 or that a metal member of a bed on which the patient lies interferes with a magnetic field radiated from the antenna 400. In the history data 241, when the reliability 2415 corresponding to specific coordinates 2413 tends to be low, it can be assumed that there is a factor (for example, a metal member) that lowers the reliability in the vicinity of the coordinate 2413.

As another use of the history data 241, for example, an operation history described in the history data 241 can be reproduced on the screen. With this configuration, since information such as what kind of operation lowers the reliability or the like can be visually obtained, it may be helpful for an operator with a relatively low proficiency.

<Sixth Embodiment>

In a sixth embodiment of the present invention, various display modes when the shape display unit 234 displays a shape reliability of the endoscope 100 on the monitor 300 will be described. The configuration of the endoscope system 1000 is similar to those in the third to fifth embodiments.

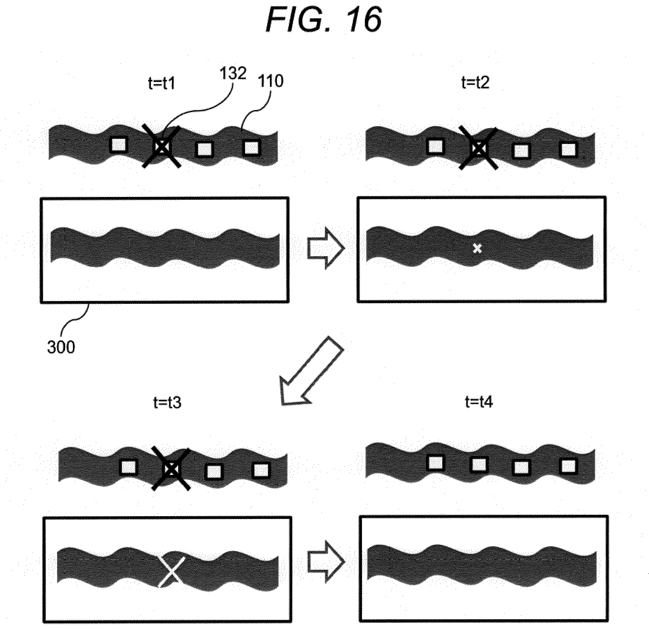

Fig. 16 illustrates an example of displaying a mark indicating that the reliability has decreased. At t = t2, the shape display unit 234 displays a cross mark at a part (which is corresponding to the shape sensor 132) where the estimation accuracy of the shape of the endoscope 100 has decreased. The size of the mark represents the degree of the lowered reliability. When a larger cross mark is displayed, the mark represents a lower reliability. Since the reliability further decreases at t = t3, the size of the cross mark is increased. Together with the mark or as a substitute for the mark, the color of the part with lowered reliability can be changed to a color different from the other parts. Furthermore, the color can be changed according to the degree of lowered reliability, and the color density and brightness can be changed. When changing the color, the color may be changed gradually with time.

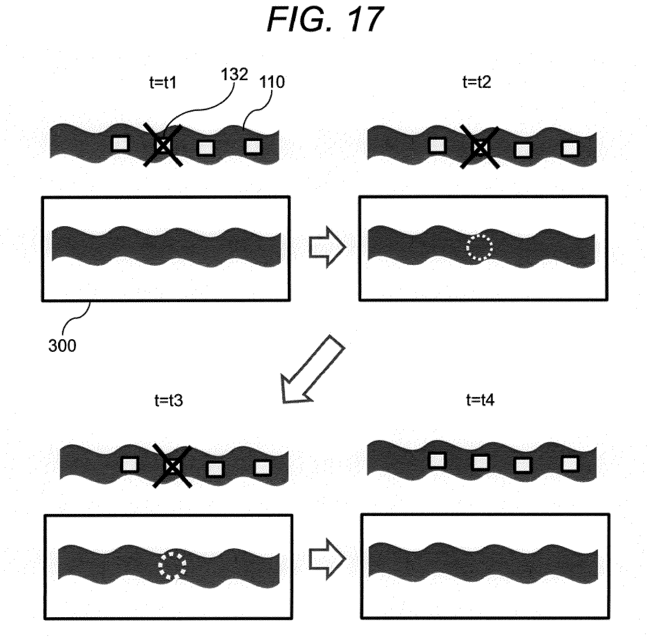

Fig. 17 illustrates an example of blinking a portion with lowered reliability. At t = t2, the shape display unit 234 displays the part corresponding to the shape sensor 132 in a blinking mode. The degree of lowered reliability can also be expressed by the frequency of blinking. For example, it is conceivable to increase the blinking frequency as the reliability is lowered.

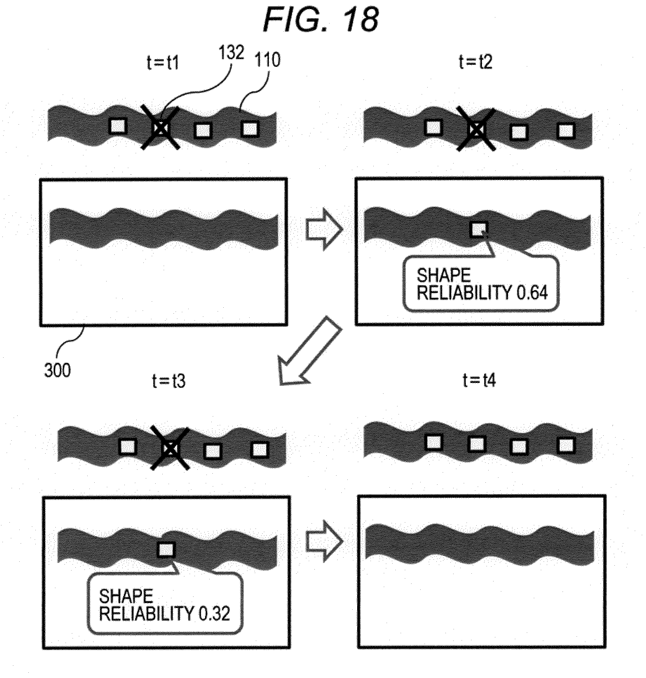

Fig. 18 illustrates an example of displaying a numerical value of reliability. The shape display unit 234 displays, on the screen, a numerical value of the reliability of the part whose reliability has decreased lower than the reliability threshold value. By indicating the part and displaying the reliability, the part where the reliability is lowered and the degree thereof can be visually recognized.

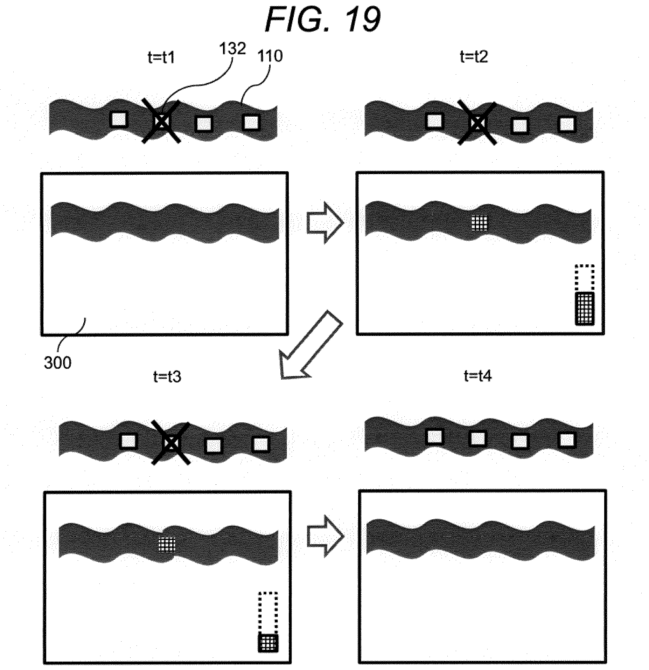

Fig. 19 illustrates an example of displaying a scale bar representing the reliability. At t = t2, the shape display unit 234 displays a mark at a part corresponding to the shape sensor 132 and also displays a scale bar having a same pattern as the mark at the lower right of the screen. The length of the scale bar represents the reliability. It is suggested that the reliability is about a half of the maximum value at t = t2. It is suggested that the reliability is about a quarter of the maximum value at t = t3.

The display modes described in the third and sixth embodiments can be used in any combination. For example, the arrow described in Fig. 10 can be used in combination with the mark described in Fig. 16. Other suitable combinations can also be used. Furthermore, as a substitute for or in addition to the reliability of the part of the endoscope 100, the reliability of the shape of the entire endoscope 100 can be displayed. In this case, the overall reliability can be calculated by an appropriate method such as averaging the reliability for each part, for example.

<Modifications of the Present Invention>

The present invention is not limited to the above embodiments and includes various modifications. For example, the above-described embodiments are described in detail to explain the present invention in an easy-to-understand manner, and is not necessarily limited to one having all the described configurations. Further, a part of the configuration of an embodiment can be replaced with a configuration of another embodiment, or a configuration of an embodiment can be added to a configuration of another embodiment. Further, it is possible to add, delete, and replace other configurations for a part of the configuration of each embodiment.

In the above embodiment, an example in which the sensor 130 is configured by a coil has been described; however, the position and orientation of the endoscope 100 may be detected by other methods. For example, an optical sensor such as a fiber bragg grating (FBG) sensor may be used. Even with this configuration, the screen display on the monitor 300 can be changed in conjunction with the reliability of the shape estimation as in the above embodiment.

In the above embodiment, the sensor 130 serves as both the position sensor and angle sensor of the endoscope 100; however, those sensors may be configured separately. For example, an angle sensor may be configured using an acceleration sensor. Further, in the above embodiments describe an example in which the four shape sensors 131 to 134 are provided; however, the number of sensors is not limited to this example.

In the above embodiments, the calculation of the reliability using Equations 1 and 2 has been described; however, other equations may be used. In other words, as long as at least one of (a) the detection accuracy of the sensor 130, (b) the shape change degree, and (c) the elapsed time is reflected as the reliability, other appropriate equations may be used. For example, only the above (a) may be reflected as the reliability. When reflecting (b) and (c) as reliability in addition to the above (a), the reliability can be obtained more accurately.

In the above embodiment, when the reliability calculation unit 233 decreases the reliability with elapsed time, instead of multiplying the inverse number of the elapsed time, the reliability is decreased according to the following function, for example: (a) Decreasing the reliability monotonously with the elapsed time, or (b) Decreasing the reliability stepwise with the elapsed time.

Examples of the site to be observed in the present invention include a respiratory organ, a digestive organ, and the like. Examples of the respiratory organ include lung, bronchi, ear, nose, and throat. Examples of digestive organs include the large intestine, small intestine, stomach, esophagus, duodenum, uterus, bladder, and the like. When the observation object has a complicated shape, it is more effective to use an operation support system by displaying a 3D image of the insertion portion of the endoscope. In particular, the large intestine has four steep bends, which is very long compared to, for example, the stomach and the like, and the practitioner is required to have a very high level of skill in operation. Further, the same problem occurs when observing the small intestine that cannot be observed without passing through the large intestine. Furthermore, the bronchi also have many branch structures, and the practitioner is required to have a very high level of skill in operation. Therefore, in the case of the endoscope for the large intestine, the endoscope for the small intestine, the endoscope for the bronchi, or the endoscope for the bladder, the effect of the present invention that can accurately assist the operation by the practitioner is more notable.

In the above embodiments, the light source 220 may be provided at the distal end of the endoscope 100. For example, a light emitting element such as an LED may be mounted on the distal end of the endoscope 100. This eliminates the need for a configuration for guiding light from the processor 200 to the distal end of the endoscope 100, thereby reducing the size of the device. In addition, the power consumption of the endoscope system 1000 can be reduced.

In the above embodiment, for example, a CCD, a CMOS, or the like can be used as the imaging device 120. It is preferable to use the imaging device 120 that can obtain a high-definition image. The high definition is, for example, one million pixels or more, more preferably two million pixels, and further preferably eight million pixels or more. Thereby, a diagnosis can be performed with high precision images with high accuracy. Further, since the practitioner operates the endoscope 100 while viewing the endoscope image on the monitor 300, it is easier to operate the distal end of the endoscope 100 by viewing the high-definition image.

In the above embodiments, the part (insertion portion) to be inserted into the observation object in the distal end of the endoscope 100 may include a passive bending part. The passive bending part is a portion that does not bend actively by operating the endoscope 100, but passively bends. When the distal end of the endoscope 100 contacts with the intestinal wall and a force is applied, this passive bending part automatically bends. Thereby, for example, a force that pushes the intestinal wall is converted into a force that advances the distal end of the endoscope 100 forward. Therefore, the patient's pain when the tip of the endoscope 100 comes into contact with the intestinal wall can be reduced. However, in the case of an endoscope having a passive bending part, the operation of the practitioner is not necessarily reflected as it is in the shape of the distal end portion of the endoscope 100, and the operation is complicated and tends to require higher skill. Therefore, in the case of the endoscope 100 including the passive bending part, the effect of the present invention that can appropriately detect the shape of the insertion site becomes more remarkable.

In the above embodiments, an insertion part with variable hardness may be employed as the insertion portion of the endoscope 100. The insertion portion may include a hardness conversion coil, a hardness conversion wire, a traction member provided at a proximal end of the wire, and a hardness change operation unit (e.g., a ring) for changing the longitudinal position of the traction member for varying the hardness. When the hardness changing wire is not pulled by the traction member, no external force is applied to the hardness changing coil, so that the hardness changing coil is in a soft state. On the other hand, when the traction member is moved by rotating a hardness changing ring, a compressive force is gradually applied to the hardness changing coil. Thereby, the hardness changes gradually so that the hardness with respect to the bending direction of the insertion portion may become high. In the case of an endoscope adopting a variable hardness, it is important to recognize the shape of the insertion portion when the hardness is low. Therefore, the effect of the present invention that can properly detect the shape of the endoscope insertion portion becomes more remarkable.

In the above embodiments, the electronic endoscope has been described as an example, but the present invention can also be applied to an endoscope using an image guide fiber or the like. In the above embodiment, a plurality of coils are arranged along the insertion portion of the endoscope 100; however, for example, a plurality of coils may be provided in a probe-like instrument, and the instrument may be mounted from a forceps opening. In that case, the coil provided in the insertion portion can be directly connected to a signal processing device different from the processor 200 without connecting via a connector portion. Thereby, the function of estimating the shape of the endoscope 100 can be made independent from the processor 200. In other words, the processor 200 that processes an image captured by the endoscope 100 and another processor that estimates the shape of the endoscope 100 can be configured separately. As in monitor 300, a monitor that displays an image captured by the endoscope 100 and a monitor that displays the shape of the endoscope 100 can be individually configured.

In the above embodiments, the magnetic field is generated by the antenna 400 arranged outside the patient's body, and the magnetic field is detected by the coil arranged inside the patient's body; however, the coil may generate a magnetic field, and the antenna 400 may detect the magnetic field.

Reference Signs List

| | |
|---|---|
| 100 | Endoscope |
| 200 | Processor |
| 210 | Receptacle |
| 220 | Light source |
| 230 | CPU |
| 231 | Captured image display unit |
| 232 | Shape estimation unit |
| 233 | Reliability calculation unit |
| 234 | Shape display unit |
| 300 | Monitor |
| 400 | Antenna |
| 1000 | Endoscope system |

**Claims**

1. An endoscope shape display device (200) that displays a shape of an endoscope (100), the endoscope shape display device (200) comprising:

   an estimation unit (232) configured to estimate the shape of the endoscope (100); a reliability calculation unit configured to calculate reliability of the shape of the endoscope estimated by the estimation unit; and
   a display unit (234) configured to display an image indicating the shape of the endoscope (100) estimated by the estimation unit (232),
   **characterized in that** the display unit (234) is configured to display, in a fade-out mode, at least a part of the image indicating the shape of the endoscope (100) according to the reliability calculated by the reliability calculation unit (233), wherein at least said part of the image gradually disappears in the fade-out mode.

2. The endoscope shape display device (200) according to claim 1, wherein
   the endoscope (100) comprises a sensor (130) configured to detect a position of the endoscope (100) and an orientation of the endoscope (100), and
   the reliability calculation unit (233) calculates the reliability based on detection accuracy of the sensor (130).

3. The endoscope shape display device (200) according to claim 2, wherein
   the reliability calculation unit (233) obtains position accuracy information indicating detection accuracy of the position of the endoscope (100) from the sensor (130) and also obtains angle accuracy information indicating detection accuracy of the orientation of the endoscope (100) from the sensor (130), and
   the reliability calculation unit (233) calculates the reliability using the position accuracy information and the angle accuracy information.

4. The endoscope shape display device (200) according to claim 3, wherein
   the reliability calculation unit (233) calculates the reliability for each part of the endoscope (100), and
   the display unit displays, in the fade-out mode, a part where the reliability is lower than a predetermined threshold value in the image of the endoscope.

5. The endoscope shape display device (200) according to claim 3, wherein
   the display unit (234) displays a position accuracy image indicating the detection accuracy of the position of the endoscope (100) and an angle accuracy image indicating the detection accuracy of the orientation of the endoscope (100), together with the shape of the endoscope (100).

6. The endoscope shape display device (200) according to claim 3, wherein
   the estimation unit (232) estimates the shape of the endoscope (100) using the position of the endoscope (100) detected by the sensor (130) and the orientation of the endoscope (100) detected by the sensor (130),
   the reliability calculation unit (233) calculates the reliability using the shape of the endoscope (100) calculated by the estimation unit (232), and
   the reliability calculation unit (233) calculates the reliability to be lower when a change speed of the shape of the endoscope (100) calculated by the estimation unit (232) is faster.

7. The endoscope shape display device (200) according to claim 1, wherein

the display unit (234) starts the fade-out mode when the reliability calculated by the reliability calculation unit (233) becomes lower than the predetermined threshold value,
the reliability calculation unit (233) calculates the reliability to be lower when elapsed time after the display unit (234) has started the fade-out mode becomes longer, and
the display unit (234) displays, in the fade-out mode, at least a part of the image indicating the shape of the endoscope (100) as the reliability becomes lower.

8. The endoscope shape display device (200) according to claim 1, wherein
the reliability calculation unit (233) calculates a change rate of the reliability with respect to time, and
when the change rate is equal to or greater than a predetermined threshold value, the display unit (234) displays an image that indicates the change rate.

9. The endoscope shape display device (200) according to claim 1, wherein
the display unit displays, in a fade-in mode, at least a part of the image indicating the shape of the endoscope (100) in conjunction with the reliability calculated by the reliability calculation unit (233).

10. An endoscope system (1000) comprising:

the endoscope shape display device (200) according to any one of claims 1 to 9; and
the endoscope (100).

11. The endoscope system (1000) according to claim 10, wherein
the estimation unit (232) comprises
a plurality of magnetic field generating elements (130, 400) provided inside the endoscope (100) or outside the endoscope (100),
a plurality of magnetic field detecting elements (130, 400) provided inside the endoscope (100) or outside the endoscope (100) where the plurality of magnetic field generating elements (130, 400) are not provided and configured to detect a magnetic field generated by the plurality of magnetic field generating elements (130, 400), and
a position estimation unit (232) configured to estimate positions of the plurality of magnetic field generating elements (130, 400) or the plurality of magnetic field detecting elements (130, 400) provided to the endoscope (100), based on the magnetic field detected by the magnetic field detecting elements (130, 400), and
the estimation unit (232) estimates the shape of the endoscope (100) based on the positions of the plurality of magnetic field generating elements (130, 400) or the plurality of magnetic field detecting elements (130, 400) estimated by the position estimation unit (232).

12. The endoscope system (1000) according to claim 10, wherein
the endoscope (100) comprises a sensor (130) configured to detect a position of the endoscope (100) and an orientation of the endoscope (100),
the reliability calculation unit (233) obtains information indicating whether or not the sensor (130) is broken, and
the display unit (234) does not perform the fade-out mode when the sensor (130) is not broken and the reliability is equal to or greater than a predetermined threshold value, and displays the image in the fade-out mode to a level that the shape of the endoscope (100) partially remains when the sensor (130) is not broken and the reliability is lower than the predetermined threshold value.

13. The endoscope system (1000) according to claim 10, wherein
the endoscope (100) comprises a sensor (130) configured to detect a position of the endoscope (100) and an orientation of the endoscope (100),
the reliability calculation unit (233) obtains information indicating whether or not the sensor (130) is broken, and
the display unit (234) displays the image in the fade-out mode to a level that the shape of the endoscope (100) partially remains when the sensor (130) is broken and the reliability is equal to or greater than a predetermined threshold value, and displays the image in the fade-out mode to a level that at least a part of the shape of the endoscope (100) completely disappears when the sensor (130) is broken and the reliability is lower than the predetermined threshold value.

**Patentansprüche**

1. Endoskopformanzeigevorrichtung (200), die eine Form eines Endoskops (100) anzeigt, wobei die Endoskopformanzeigevorrichtung (200) umfasst:

eine Schätzeinheit (232), die ausgebildet ist, um die Form des Endoskops (100) zu schätzen;
eine Zuverlässigkeitsberechnungseinheit, die ausgebildet ist, um die von der Schätzeinheit geschätzte Zuverlässigkeit der Form des Endoskops zu berechnen; und
eine Anzeigeeinheit (234), die ausgebildet ist, um ein Bild, das die von der Schätzeinheit (232) geschätzte Form des Endoskops (100) angibt, anzuzeigen,
**dadurch gekennzeichnet, dass** die Anzeigeeinheit (234) ausgebildet ist, um in einem Ausblendmodus zumindest einen Teil des Bildes, der die Form des Endoskops (100) gemäß der von der Zuverlässigkeitsberechnungseinheit (233) berechneten Zuverlässigkeit angibt, anzuzeigen, wobei zumindest der genannte Teil des Bildes in dem Ausblendmodus nach und nach verschwindet.

2. Endoskopformanzeigevorrichtung (200) nach Anspruch 1, wobei das Endoskop (100) einen Sensor (130) umfasst, der ausgebildet ist, um eine Position des Endoskops (100) und eine Ausrichtung des Endoskops (100) zu erfassen, und
die Zuverlässigkeitsberechnungseinheit (233) die Zuverlässigkeit basierend auf der Erfassungsgenauigkeit des Sensors (130) berechnet.

3. Endoskopformanzeigevorrichtung (200) nach Anspruch 2, wobei die Zuverlässigkeitsberechnungseinheit (233) die Positionsgenauigkeitsinformation, die die Erfassungsgenauigkeit der Position des Endoskops (100) angibt, aus dem Sensor erhält und ferner eine Winkelgenauigkeitsinformation, die die Erfassungsgenauigkeit der Ausrichtung des Endoskops (100) angibt, aus dem Sensor (130) erhält, und
die Zuverlässigkeitsberechnungseinheit (233) die Zuverlässigkeit unter Verwendung der Positionsgenauigkeitsinformation und der Winkelgenauigkeitsinformation berechnet.

4. Endoskopformanzeigevorrichtung (200) nach Anspruch 3, wobei die Zuverlässigkeitsberechnungseinheit (233) die Zuverlässigkeit für jeden Teil des Endoskops (100) berechnet, und
die Anzeigeeinheit in dem Ausblendmodus einen Teil, in dem die Zuverlässigkeit niedriger als ein vorbestimmter Schwellenwert ist, in dem Bild des Endoskops anzeigt.

5. Endoskopformanzeigevorrichtung (200) nach Anspruch 3, wobei die Anzeigeeinheit (234) ein Positionsgenauigkeitsbild anzeigt, das die Erfassungsgenauigkeit der Position des Endoskops (100) angibt, sowie ein Winkelgenauigkeitsbild, das die Erfassungsgenauigkeit der Ausrichtung des Endoskops (100) angibt, zusammen mit der Form des Endoskops (100).

6. Endoskopformanzeigevorrichtung (200) nach Anspruch 3, wobei die Schätzeinheit (232) die Form des Endoskops (100) unter Verwendung der von dem Sensor (130) erfassten Position des Endoskops (100) und der von dem Sensor (130) erfassten Ausrichtung des Endoskops (100) schätzt, und
die Zuverlässigkeitsberechnungseinheit (233) die Zuverlässigkeit unter Verwendung der Form des Endoskops (100), die von der Schätzeinheit (232) berechnet wurde, berechnet, und
die Zuverlässigkeitsberechnungseinheit (233) die Zuverlässigkeit als niedriger berechnet, wenn eine Änderungsgeschwindigkeit der Form des Endoskops (100), die von der Schätzeinheit (232) berechnet wird, schneller ist.

7. Endoskopformanzeigevorrichtung (200) nach Anspruch 1, wobei die Anzeigeeinheit (234) den Ausblendmodus startet, wenn die von der Zuverlässigkeitsberechnungseinheit (233) berechnete Zuverlässigkeit niedriger als der vorbestimmte Schwellenwert wird,
die Zuverlässigkeitsberechnungseinheit (233) die Zuverlässigkeit als niedriger berechnet, wenn die abgelaufene Zeit, nachdem die Anzeigeeinheit (234) den Ausblendmodus gestartet hat, länger wird, und die Anzeigeeinheit (234) in dem Ausblendmodus zumindest einen Teil des Bildes anzeigt, der die Forms des Endoskops (100) angibt, wenn die Zuverlässigkeit niedriger wird.

8. Endoskopformanzeigevorrichtung (200) nach Anspruch 1 wobei die Zuverlässigkeitsberechnungseinheit (233) eine Änderungsrate der Zuverlässigkeit in Bezug auf die Zeit berechnet, und
wenn die Änderungsrate gleich oder größer als ein vorbestimmter Schwellenwert ist, die Anzeigeeinheit (234) ein Bild anzeigt, das die Änderungsrate angibt.

9. Endoskopformanzeigevorrichtung (200) nach Anspruch 1, wobei die Anzeigeeinheit in einem Ausblendmodus zumindest einen Teil des Bildes anzeigt, der die Form des Endoskops (100) angibt, in Verbindung mit der von der Zuverlässigkeitsberechnungseinheit (233) berechneten Zuverlässigkeit.

10. Endoskopsystem (1000), umfassend:

die Endoskopformanzeigevorrichtung (200) nach einem der Ansprüche 1 bis 9; und
das Endoskop (100).

11. Endoskopsystem (1000) nach Anspruch 10, wobei die Schätzeinheit (232) umfasst
eine Vielzahl von Magnetfelderzeugungselementen (130, 400), die innerhalb des Endoskops (100) oder außerhalb des Endoskops (100) vorgesehen sind,
eine Vielzahl von Magnetfelderfassungselementen (130) 400, die innerhalb des Endoskops (100) oder außerhalb des Endoskops (100) dort vorgesehen ist, wo die Vielzahl von Magnetfelderzeugungselementen (130, 400) nicht vorgesehen ist, und ausgebildet ist, um ein von der Vielzahl von Magnetfelderzeugungselementen (130, 400) erzeugtes Magnetfeld zu erfassen, und
eine Positionsschätzeinheit (232), die ausgebildet ist, um Positionen der Vielzahl von Magnetfelderzeugungselementen (130, 400) oder der Vielzahl von Magnetfelderfassungselementen (130, 400), die an dem Endoskop (100) vorgesehen ist, basierend auf dem Magnetfeld, das von den Magnetfelderfassungselementen (130, 400) erfasst wird, zu schätzen, und
die Schätzeinheit (232) die Form des Endoskops (100) basierend auf den Positionen der Vielzahl von Magnetfelderzeugungselementen (130, 400) oder der Vielzahl von Magnetfelderfassungselementen (130, 400), die von der Positionsschätzeinheit geschätzt werden, schätzt.

12. Endoskopsystem (1000) nach Anspruch 10, wobei das Endoskop (100) einen Sensor (130) umfasst, der ausgebildet ist, um eine Position des Endoskops (100) und eine Ausrichtung des Endoskops (100) zu erfassen,
die Zuverlässigkeitsberechnungseinheit (233) eine Information, die angibt, ob der Sensor (130) defekt ist oder nicht, ermittelt, und
die Anzeigeeinheit (234) den Ausblendmodus nicht ausführt, wenn der Sensor (130) nicht defekt und die Zuverlässigkeit gleich oder größer als ein vorbestimmter Schwellenwert ist, und das Bild in dem Ausblendmodus bis zu einer Höhe anzeigt, dass die Form des Endoskops (100) teilweise erhalten bleibt, wenn der Sensor (130) nicht defekt und die Zuverlässigkeit niedriger als der vorbestimmte Schwellenwert ist.

13. Endoskopsystem (1000) nach Anspruch 10, wobei das Endoskop (100) einen Sensor (130) umfasst, der ausgebildet ist, um eine Position des Endoskops (100) und eine Ausrichtung des Endoskops (100) zu erfassen,
die Zuverlässigkeitsberechnungseinheit (233) eine Information ermittelt, die angibt, ob der Sensor (130) defekt ist oder nicht, und
die Anzeigeeinheit (233) das Bild in dem Ausblendmodus bis zu einer Höhe anzeigt, dass die Form des Endoskops (100) teilweise erhalten bleibt, wenn der Sensor (130) defekt und die Zuverlässigkeit gleich oder größer als einer vorbestimmter Schwellenwert ist, und das Bild in dem Ausblendmodus bis zu einer Höhe anzeigt, dass zumindest ein Teil der Form des Endoskops (100) vollständig verschwindet, wenn der Sensor (130) defekt und die Zuverlässigkeit niedriger als der vorbestimmte Schwellenwert ist.

**Revendications**

1. Dispositif d'affichage de forme d'endoscope (200) qui affiche une forme d'un endoscope (100), le dispositif d'affichage de forme d'endoscope (200) comprenant :

une unité d'estimation (232) configurée pour estimer la forme de l'endoscope (100) ;
une unité de calcul de fiabilité configurée pour calculer une fiabilité de la forme de l'endoscope estimée par l'unité d'estimation ; et
une unité d'affichage (234) configurée pour afficher une image indiquant la forme de l'endoscope (100) estimée par l'unité d'estimation (232),
**caractérisé en ce que**
l'unité d'affichage (234) est configurée pour afficher, dans un mode de fondu sortant, au moins une partie de l'image indiquant la forme de l'endoscope (100) selon la fiabilité calculée par l'unité de calcul de fiabilité (233), dans lequel ladite partie de l'image disparaît progressivement dans le mode de fondu sortant.

2. Dispositif d'affichage de forme d'endoscope (200) selon la revendication 1, dans lequel

l'endoscope (100) comprend un capteur (130) configuré pour détecter une position de l'endoscope (200) et une orientation de l'endoscope (100), et

l'unité de calcul de fiabilité (233) calcule la fiabilité sur la base d'une précision de détection du capteur (130).

3. Dispositif d'affichage de forme d'endoscope (200) selon la revendication 2, dans lequel

l'unité de calcul de fiabilité (233) obtient des informations de précision de position indiquant une précision de détection de la position de l'endoscope (100) à partir du capteur (130) et obtient également des informations de précision d'angle indiquant une précision de détection de l'orientation de l'endoscope (100) à partir du capteur (130), et

l'unité de calcul de fiabilité (233) calcule la fiabilité en utilisant les informations de précision de position et les informations de précision d'angle.

4. Dispositif d'affichage de forme d'endoscope (200) selon la revendication 3, dans lequel

l'unité de calcul de fiabilité (233) calcule la fiabilité pour chaque partie de l'endoscope (100), et

l'unité d'affichage affiche, dans le mode de fondu sortant, une partie où la fiabilité est inférieure à une valeur de seuil prédéterminée dans l'image de l'endoscope.

5. Dispositif d'affichage de forme d'endoscope (200) selon la revendication 3, dans lequel

l'unité d'affichage (234) affiche une image de précision de position indiquant la précision de détection de la position de l'endoscope (100) et une image de précision d'angle indiquant la précision de détection de l'orientation de l'endoscope (100), ensemble avec la forme de l'endoscope (100).

6. Dispositif d'affichage de forme d'endoscope (200) selon la revendication 3, dans lequel

l'unité d'estimation (232) estime la forme de l'endoscope (100) en utilisant la position de l'endoscope (100) détectée par le capteur (130) et l'orientation de l'endoscope (100) détectée par le capteur (130),

l'unité de calcul de fiabilité (233) calcule la fiabilité en utilisant la forme de l'endoscope (100) calculée par l'unité d'estimation (232), et

l'unité de calcul de fiabilité (233) calcule la fiabilité pour être inférieure lorsqu'une vitesse de changement de la forme de l'endoscope (100) calculée par l'unité d'estimation (232) est plus rapide.

7. Dispositif d'affichage de forme d'endoscope (200) selon la revendication 1, dans lequel

l'unité d'affichage (234) démarre le mode de fondu sortant lorsque la fiabilité calculée par l'unité de calcul de fiabilité (233) devient inférieure à la valeur de seuil prédéterminée,

l'unité de calcul de fiabilité (233) calcule la fiabilité pour être inférieure lorsque le temps écoulé après que l'unité d'affichage (234) a commencé le mode de fondu de sortie devient plus long, et

l'unité d'affichage (234) affiche, dans le mode de fondu sortant, au moins une partie de l'image indiquant la forme de l'endoscope (100) lorsque la fiabilité devient inférieure.

8. Dispositif d'affichage de forme d'endoscope (200) selon la revendication 1, dans lequel

l'unité de calcul de fiabilité (233) calcule un taux de variation de la fiabilité par rapport au temps, et

lorsque le taux de changement est égal ou supérieur à une valeur de seuil prédéterminée, l'unité d'affichage (234) affiche une image qui indique le taux de changement.

9. Dispositif d'affichage de forme d'endoscope (200) selon la revendication 1, dans lequel

l'unité d'affichage affiche, dans un mode de fondu entrant, au moins une partie de l'image indiquant la forme de l'endoscope (100) conjointement avec la fiabilité calculée par l'unité de calcul de fiabilité (233).

10. Système d'endoscope (1000) comprenant :

le dispositif d'affichage de forme d'endoscope (200) selon l'une quelconque des revendications 1 à 9 ; et
l'endoscope (100).

11. Système d'endoscope (1000) selon la revendication 10, dans lequel

l'unité d'estimation (232) comprend
une pluralité d'éléments générateurs de champ magnétique (130, 400) fournis à l'intérieur de l'endoscope (100) ou à l'extérieur de l'endoscope (100),

une pluralité d'éléments de détection de champ magnétique (130, 400) fournis à l'intérieur de l'endoscope (100) ou à l'extérieur de l'endoscope (100) où la pluralité d'éléments générateurs de champ magnétique (130, 400) ne sont pas fournis et configurés pour détecter un champ magnétique généré par la pluralité d'éléments générateurs de champ magnétique (130, 400), et
une unité d'estimation de position (232) configurée pour estimer des positions de la pluralité d'éléments générateurs de champ magnétique (130, 400) ou de la pluralité d'éléments de détection de champ magnétique (130, 400) fournis à l'endoscope (100), sur la base du champ magnétique détecté par les éléments de détection de champ magnétique (130, 400), et
l'unité d'estimation (232) estime la forme de l'endoscope (100) sur la base des positions de la pluralité d'éléments générateurs de champ magnétique (130, 400) ou de la pluralité d'éléments de détection de champ magnétique (130, 400) estimées par l'unité d'estimation de position (232).

12. Système d'endoscope (1000) selon la revendication 10, dans lequel
l'endoscope (100) comprend un capteur (130) configuré pour détecter une position de l'endoscope (100) et une orientation de l'endoscope (100),
l'unité de calcul de fiabilité (233) obtient des informations indiquant si le capteur (130) est cassé ou non, et
l'unité d'affichage (234) n'effectue pas le mode de fondu sortant lorsque le capteur (130) n'est pas cassé et la fiabilité est égale ou supérieure à une valeur de seuil prédéterminée, et affiche l'image dans le mode de fondu sortant à un niveau tel que la forme de l'endoscope (100) reste partiellement lorsque le capteur (130) n'est pas cassé et la fiabilité est inférieure à la valeur de seuil prédéterminée.

13. Système d'endoscope (1000) selon la revendication 10, dans lequel
l'endoscope (100) comprend un capteur (130) configuré pour détecter une position de l'endoscope (100) et une orientation de l'endoscope (100),
l'unité de calcul de fiabilité (233) obtient des informations indiquant si le capteur (130) est cassé ou non, et
l'unité d'affichage (234) affiche l'image dans le mode de fondu sortant à un niveau tel que la forme de l'endoscope (100) reste partiellement lorsque le capteur (130) est cassé et la fiabilité est égale ou supérieure à une valeur de seuil prédéterminée, et affiche l'image dans le mode de fondu sortant à un niveau tel qu'au moins une partie de la forme de l'endoscope (100) disparaît complètement lorsque le capteur (130) est cassé et la fiabilité est inférieure à la valeur de seuil prédéterminée.

FIG. 1

EP 3 607 870 B1

# FIG. 2

230

CPU

CAPTURED IMAGE DISPLAY UNIT — 231

SHAPE ESTIMATION UNIT — 232

RELIABILITY CALCULATION UNIT — 233

SHAPE DISPLAY UNIT — 234

FIG. 3

# FIG. 4

```
                        ┌─────────────┐
                        │    START    │
                        └─────────────┘
                               │
                        ┌─────────────────┐  S401
                        │ OBTAIN SENSOR DATA │
                        └─────────────────┘
                               │
                         ╱─────────────╲  S402
              N    ╱ IS DETECTION ╲    Y
          ◄───────╱ ACCURACY LOW? ╲───────┐
                  ╲                ╱        │
                   ╲──────────────╱         │
                                            ▼
                                    ╱─────────────╲  S407
                                   ╱ IS SENSOR BROKEN? ╲──────────────┐ Y
                                    ╲                  ╱               │
                                     ╲────────────────╱                │
                                         │ N                           │
```

S403 — ESTIMATE POSITION AND ORIENTATION
S404 — ESTIMATE SHAPE
S405 — CALCULATE RELIABILITY
S406 — NORMAL DISPLAY

S408 — ESTIMATE POSITION AND ORIENTATION
S409 — ESTIMATE SHAPE
S410 — CALCULATE RELIABILITY
S411 — IS RELIABILITY HIGH?
S412 — FADE-OUT TO SOME LEVEL

S413 — ESTIMATE POSITION AND ORIENTATION
S414 — ESTIMATE SHAPE
S415 — CALCULATE RELIABILITY
S416 — IS RELIABILITY HIGH?
S417 — FADE-OUT TO COMPLETE LEVEL

END

## FIG. 5

```
                    ┌─────────────────┐
                    │      START      │
                    └────────┬────────┘
                             │
                             ▼
            ┌────────────────────────────────┐    S501
            │   OBTAIN DETECTION ACCURACY     │
            └────────────────┬───────────────┘
                             │
                             ▼
            ┌────────────────────────────────┐    S502
            │  CALCULATE SHAPE CHANGE DEGREE  │
            └────────────────┬───────────────┘
                             │
                             ▼                       S503
                        ╱──────────╲
                       ╱ IS ELAPSED ╲
                      ╱  TIME BEING   ╲──── Y
                      ╲  COUNTED?     ╱            S504
                       ╲──────────╱        ┌──────────────────────────┐
                            │ N            │ MULTIPLY (1/ELAPSED TIME) │
                            │              └──────────────┬───────────┘
                            │                             │
                            │                             ▼          S505
                            │                        ╱──────────╲
                            │                       ╱  IS SENSOR  ╲──── N
                            │                       ╲  BROKEN?    ╱        S506
                            │                        ╲──────────╱    ┌─────────────────────┐
                            │                             │ Y        │ ROUND UP RELIABILITY │
                            │                             │          └──────────┬──────────┘
                            │                             │◄────────────────────┘
                            │                             ▼                S507
                            │                    ┌──────────────────────┐
                            │                    │  UPDATE ELAPSED TIME │
                            │                    └──────────┬───────────┘
                            │◄──────────────────────────────┘
                            ▼
                    ┌─────────────────┐
                    │       END       │
                    └─────────────────┘
```

EP 3 607 870 B1

## FIG. 6

t=t3    132   110

t=t4

300

t=t5

EP 3 607 870 B1

# FIG. 7

t=t3  132  110

t=t4

300

t=t5

t=t6

# FIG. 8

t=t1

132  110

t=t2

DETECTION ACCURACY = 0.7
SHAPE CHANGE DEGREE = 0.1

300

t=t3

DETECTION ACCURACY = 0.7
SHAPE CHANGE DEGREE = 0.1

t=t4

EP 3 607 870 B1

# FIG. 9

t=t1                                t=t2

(a)

SHAPE RELIABILITY ⬇

(b)

SHAPE RELIABILITY ⬇

(c)

SHAPE RELIABILITY ⬆

## FIG. 10

t=t1

132  110

t=t2

300

SHAPE
RELIABILITY

t=t3

t=t4

SHAPE
RELIABILITY

## FIG. 11

START

↓

OBTAIN SENSOR DATA — S1101

↓

ESTIMATE POSITION AND ORIENTATION — S1102

↓

ESTIMATE SHAPE — S1103

↓

CALCULATE RELIABILITY — S1104

↓

S1105

LOWER THAN THRESHOLD VALUE?

N ← → Y

S1106 — NORMAL DISPLAY

S1107 — DISPLAYED WITH RELIABILITY IMAGE

↓

END

## FIG. 12

```
                    ┌──────────────┐
                    │    START     │
                    └──────┬───────┘
                           │
                           ▼
          ┌──────────────────────────────┐      S1201
          │  OBTAIN DETECTION ACCURACY    │
          └──────────────┬────────────────┘
                         │
                         ▼
          ┌──────────────────────────────┐      S1202
          │ CALCULATE SHAPE CHANGE DEGREE │
          └──────────────┬────────────────┘
                         │
                         ▼            S1203
                    ╱──────────╲
                   ╱  IS ELAPSED ╲          Y
                  ╱   TIME BEING   ╲──────────────────┐
                  ╲   COUNTED?     ╱                   │
                   ╲──────────────╱                    ▼
                         │ N              ┌────────────────────────────┐  S1204
                         │                │ MULTIPLY (1/ELAPSED TIME)  │
                         │                └─────────────┬──────────────┘
                         │                              │
                         │                              ▼          S1205
                         │                ┌────────────────────────────┐
                         │                │   UPDATE ELAPSED TIME       │
                         │                └─────────────┬──────────────┘
                         │                              │
                         │◄─────────────────────────────┘
                         ▼
                  ┌──────────────┐
                  │     END      │
                  └──────────────┘
```

EP 3 607 870 B1

FIG. 13

EP 3 607 870 B1

# FIG. 14

EP 3 607 870 B1

## FIG. 15

241

| DATE AND TIME<br>2411 | SENSOR<br>NUMBER<br>2412 | COORDINATES<br>2413 | ORIENTATION<br>2414 | RELIABILITY<br>2415 |
|---|---|---|---|---|
| 20170101 09:00:00 | 1 | x1, y1, z1 | $\theta1, \phi1$ | 0.78 |

.
.

# FIG. 16

FIG. 17

FIG. 18

# FIG. 19

**EP 3 607 870 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002325721 A